# EUROPEAN PATENT SPECIFICATION

(11) **EP 0 995 414 B1**
(45) Date of publication and mention of the grant of the patent: **03.09.2003**
(21) Application number: 98830565.2
(22) Date of filing: 28.09.1998
(51) Int. Cl.: A61F 13/15, B26F 1/10

(54) **An apertured film combined with a fibrous web, method of fabrication and absorbent article comprising said film**
Perforierter Film kombiniert mit einer Faserstoffbahn, Verfahren zu seiner Herstellung sowie dieser Film enthaltender saugfähiger Einwegartikel
Film perforé combiné avec une bande fibreuse, procédé de fabrication et article absorbant comprenant ledit film

(43) Date of publication of application: 26.04.2000
(73) Proprietor: PANTEX S.r.l., I-51031 Agliana, Pistoia (IT)
(72) Inventor: Bargiacchi, Franco, 51100 Pistoia (IT)
(74) Representative: Mannucci, Michele

(56) References cited:
- EP-A- 0 403 187
- EP-A- 0 598 970
- EP-A- 0 738 505
- US-A- 4 781 962
- US-A- 5 704 101

## Description

### Technical Field

The present invention relates to a perforated laminar product, and more particularly to a three-dimensional perforated thermoplastic film, particularly suitable for use in the production of absorbent articles, such as female sanitary napkins, diapers for babies, diapers for incontinent persons and the like.

The present invention also relates to an absorbent article of the type comprising a top sheet which is permeable to liquids, a back sheet which is non-permeable to liquids, an absorbent mass which is arranged between the top sheet and the back sheet, and an acquisition layer arranged between the absorbent mass and the top sheet.

### State of the Art

In the production of absorbent articles, such as for example female sanitary napkins, diapers for babies, diapers for incontinent persons and the like, perforated plastic films which form the top sheet of the article and which come into contact with the user's skin are frequently used. With regard to the design of these films it is important that they should provide the user with a sensation of well-being, preventing the skin from coming into contact with the moisture which is absorbed by the absorbent mass forming the core of the article.

For this purpose so-called three-dimensional perforated thermoplastic films have been made, namely films which have been treated mechanically in such a way that their overall thickness is much greater than the thickness of the plastic base material, typically even ten times greater. This may be obtained in various ways. According to a particularly advantageous technique, described in EP-A-0,598,970, a two-dimensional film (i.e. with a limited thickness, typically a few tens of micrometers) is made to pass between two counter-rotating cylinders, one of which is smooth, while the other one has a series of protuberances. The latter rotates at a peripheral speed which is greater than that of the smooth cylinder, for example with a speed difference of 15-25%. The film is fed into the nip formed between the two cylinders at a speed which is lower than the peripheral speed of the cylinder equipped with protuberances. In this way, perforation of the film and deformation of the base material forming it are achieved, together with the formation of lugs around the perforations, which project outside the plane of the film, on the opposite side with respect to the protuberances which have produced the perforations. The overall thickness of the material which is thus obtained, defined by the lugs produced as a result of plastic deformation, is even ten times greater than the initial thickness and typically of the order of several hundreds of micrometers.

Other methods for the formation of three-dimensional thermoplastic films are described, for example, in WO-A-9,312,749, EP-A-0,018,020, US-A-3,929,135 and other documents mentioned in these publications. According to some of these methods, the lugs consist of frustoconical walls or walls which form, together with the base film, U-shaped bridge-pieces. In general, however, all these methods involve the formation, by means of the base material forming the thermoplastic film, of lugs which project from the original plane of the film around the perforations produced using different techniques.

In the production of absorbent articles, a portion of perforated film of this type is used in order to form the top sheet of the article which is welded peripherally to an impermeable back sheet, with the lugs obtained by the plastic deformation of the film being directed toward the inside of the article. The absorbent mass, if necessary composed of several different layers, is arranged between the two - upper and lower - layers. The lugs produced by the plastic deformation of the perforated film keep the external surface of the perforated film at a certain distance from the internal absorbent mass, in order to avoid contact between the moist part of the article and the user's skin. The lugs form, moreover, a certain obstacle to the reverse flow of the body liquid which the article is intended to absorb.

Usually, between the top sheet and the absorbent mass a so-called acquisition layer is arranged, said layer being formed by hydrophilic fibers or hydrophobic fibers treated so as to assume hydrophilic properties, typically in the form of a non-woven fabric. Preferably fibers of polypropylene, polyethylene, nylon®, polyester, rayon®, cellulose acetate and the like are used for this purpose. The object of the acquisition layer is to drain away rapidly the liquid which penetrates inside the article through the openings of the film and convey it rapidly toward the underlying absorbent mass. By so doing, the liquid is also distributed horizontally so that it is absorbed in a uniform manner by the underlying absorbent mass, instead of in an irregular manner, with greater absorption in the region of the openings of the overlying film.

Usually the acquisition layer is placed against the top sheet or glued thereto. When the layer is simply placed against it, there is the drawback that the plastic film tends to become detached from the absorbent mass, adhering to the user's skin and creating considerable discomfort. The use of adhesives to keep the top sheet adherent to the internal fibers has considerable drawbacks, including the risk of detachment, the tendency to give the user a sensation of stickiness, and the risk of worsening of the conditions with which the body liquid flows toward the inside of the article, on account of the obstacle which the adhesive creates in the vicinity of the openings in the film.

WO-A-9,311,725 describes a different method for the production of an absorbent article, comprising a top sheet consisting of a three-dimensional perforated film and an acquisition layer. According to this known technique, a web of non-woven fabric is joined to the perforated three-dimensional film by means of spot-welding. At the welding points there is total fusion of the film which has a melting temperature lower than the melting temperature of the fibers forming the acquisition layer.

This method, although providing advantages compared to the use of adhesives for combining the acquisition layer with the three-dimensional perforated film, has considerable drawbacks. Firstly it requires a specific production stage, during which the acquisition layer is applied to the three-dimensional perforated film, with the consequent need to subject the film to two processing steps. This increases the production costs and may subject the film to a high degree of stress.

Moreover, the presence of the welding spots alters the distribution of the liquid flow holes. The density of the open zones of the film is a critical parameter for manufacture of a film, which provides the absorbent article with the desired qualities of absorption and comfort for the user. The need to add openings resulting from the melting together of the film and acquisition layer makes the design of the perforation pattern more critical.

Moreover, in the relatively extensive melting zones, where the perforated film and the acquisition layer are joined together, the barrier effect provided by the three-dimensional nature of the perforated film is partially lost.

US-A-4,781,962 discloses a method for the production of a composite article consisting of a plastic film joined to a web of fibers, to be used in the production of absorbent articles. In this case the product is obtained by means of calendering of an intact film and a web of non-woven fabric. The calendering causes perforation of the film, the thickness of which is not increased with respect to the thickness of the intact film, while in the vicinity of the holes of the film the non-woven fabric increases in density owing to the compression exerted in the calender. This laminar product is used in the manufacture of the top sheet of diapers and sanitary napkins, arranging the side lined with non-woven fabric toward the outside, i.e. in contact with the user's skin. The layer of fibers therefore does not form the acquisition layer of the absorbent article. Moreover, the film is not three-dimensional.

EP-A-0,403,187 describes a method for joining a previously perforated film to a web of non-woven fabric. In this case the film is first perforated and then joined to the non-woven fabric by means of welding spots.

WO-A-9,424,354 describes a method for obtaining a laminar article consisting of the joining of a web of non-woven fabric to a three-dimensional perforated plastic film. According to this method, the web of non-woven fabric is laid on the film and the whole surface of the film is heated up to the melting temperature. Perforation is obtained pneumatically by feeding the plastic film around a perforated roll inside which a vacuum is created. The web of non-woven fabric adheres substantially over the entire surface of the film and is therefore compact and not soft. The method is used mainly to provide the external surface of the plastic film with a textile appearance and not in order to provide an acquisition layer adhering to the perforated film.

US-A-5704101, on which the preamble of claims 1 and 11 are based, discloses a method for producing a perforated plastic film combined with a fiber layer and a product obtained with such a method. According to this known method, the plastic film is combined with a non-woven textile web which is made of a consolidated fiber web. The non-woven web is unwound from a roll and placed on top of the film prior to passing the two materials through the nip between an anvil roll and a pattern roll.

### Objects and Summary of the Invention

The object of the present invention is to propose a more simple, more economical and more efficient method for joining the perforated three-dimensional film to the acquisition layer.

The object of the present invention is also that of providing an absorbent article of the type mentioned above which is more economical to manufacture and more efficient and more pleasing to use.

These and further objects and advantages, which will become obvious to persons skilled in the art from a reading of the text which follows, are obtained with a method for the production of a three-dimensional, perforated, thermoplastic film, characterized by the fact of superimposing an intact film and a web of fibers and perforating said film so as to cause, in the region of perforations produced in the film, mutual adhesion between said film and said web.

The invention is based on the observation of the fact that it is possible to cause welding of the web of fibers to the film during perforation of the latter without causing compacting and an increase in density of the fiber and keeping the latter sufficiently free and soft to form an acquisition layer in an absorbent article. The density of the fibers remains substantially constant even after perforation. A high degree of softness is obtained when the web is a web of fibers which are unconsolidated.

In practice the three-dimensional thermoplastic film is obtained, in a manner known per se, by forming lugs of plastic material projecting from the plane of the film. According to the invention, during this perforation step with formation of lugs projecting from the plane of the film, the web is attached to the film by means of melting of the fibers and/or the film in the region of said lugs.

It has been surprisingly found that the perforation method described in EP-A-0,598,970 is particularly suitable for the production of a three-dimensional perforated film with a web of unconsolidated fibers joined thereto in the region of the perforations, in which this web retains optimum properties of low density, softness and delicacy. Therefore, according to a particularly advantageous embodiment, the method is characterized by the fact of perforating said film by means of a pair of counter-rotating cylinders which are pressed against one another and one of which has a substantially smooth surface and the other a plurality of protuberances, the cylinder equipped with protuberances rotating at a peripheral speed which is greater than the peripheral speed of the other cylinder and greater than the speed of feeding of the film and web between said cylinders, opposite said protuberances the film being perforated with the formation of lugs of material which are partially detached from said film and the fibers of said web being welded to said lugs.

It has been surprisingly found that the fibers of the web remain substantially free and soft also in the region of the perforations, i.e. where they are compressed by the protuberances of the cylinder.

According to a preferred embodiment of the invention, the fibers are thermoplastic fibers, even though in general terms it is possible to consider using also non-thermoplastic fibers, causing adhesion thereof to the thermoplastic film by means of melting of the latter in the region of the perforations and incorporating the fibers of the web in the fused mass.

Preferably at least some of the fibers have a melting temperature which is lower than the melting temperature of said thermoplastic film. Advantageously it is possible to use bi-component fibers consisting of a shell and a core, said shell and core having different melting temperatures, the shell having a melting temperature lower than that of the core. Preferably the web is formed by a mixture of bi-component fibers and single-component fibers having a melting temperature which is higher than the melting temperature of the shell of the bi-component fibers. In this way, during perforation, melting of a very small percentage of the material forming the fibers occurs, with consequent maintaining of an even greater degree of softness of the web after joining to the film.

According to a particularly advantageous embodiment of the invention, the thermoplastic film has a layered structure, with at least one layer having a lower melting temperature and one layer having a higher temperature. Typically the film has an odd number of layers, for example three or five layers. In this case the internal layer has a higher melting temperature and allows the necessary consistency to be maintained during the whole perforation treatment.

The invention also relates to a composite laminar material comprising a three-dimensional thermoplastic film equipped with perforations and a web of fibers joined thereto, characterized in that said web of fibers is attached by means of welding to said film in the region of said perforations.

The invention also relates to an absorbent article using a laminar material of this type for forming the top sheet and the acquisition layer.

Further advantageous features and embodiments of the method and the product according to the invention are described in the accompanying claims.

### Brief Description of the Drawings

The invention will be better understood with reference to the description and the attached drawing which shows a practical non-limiting embodiment of the invention. More particularly, in said drawing:
Fig. 1 shows a diagram of a plant for implementing the method according to the invention;
Fig. 2 shows an enlarged plan view of a film according to the invention, from the external side;
Fig. 3 shows a cross section along III-III of Fig. 1;
Fig. 4 shows a cross section along IV-IV of Fig. 3;
Fig. 5 shows a cross section through an absorbent article made with the product according to the invention;
Fig. 6 shows an enlarged photograph of a cross section of the film joined to the web of fibers; and
Figs. 7 and 8 show two enlarged photographs with a view of the film joined to the web of fibers, respectively from the film side and from the web side.

### Detailed Description of the Preferred Embodiment

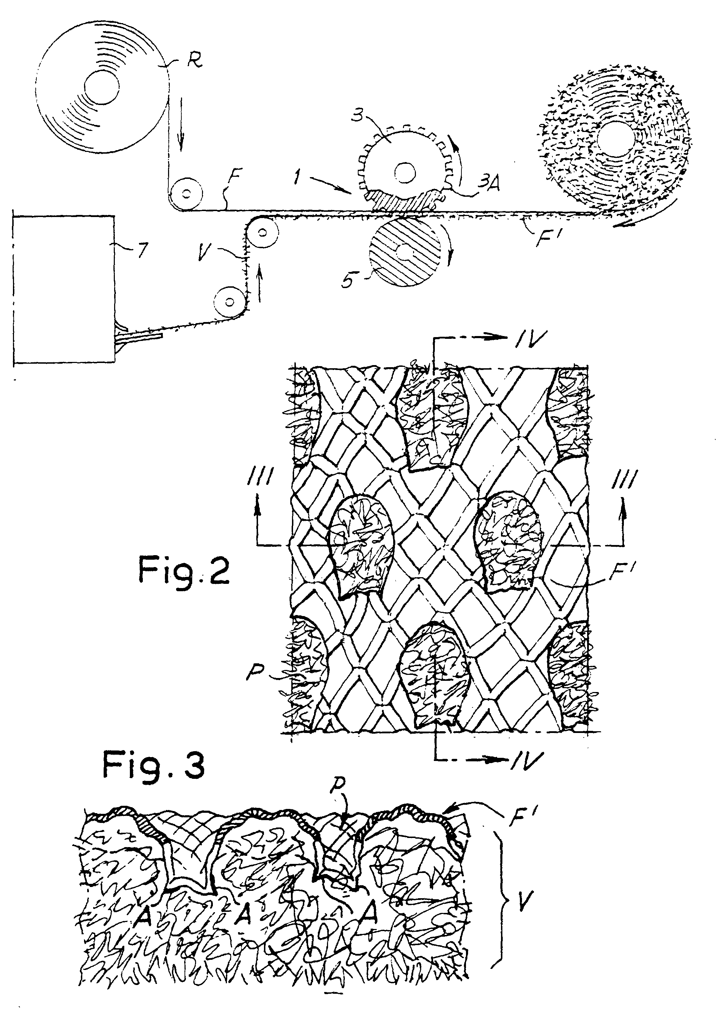

Fig. 1 shows schematically an example of a plant for the production of a film joined to an acquisition layer using the method according to the invention. The plastic film F which is intact, i.e. not yet perforated, is wound onto a roll R from where it is unwound so as to be supplied to a calender 1, comprising a first lower cylinder 3 equipped with a plurality of protuberances 3A, shown for the sake of simplicity on a much larger scale than the real size in relation to the diameter of the cylinder. The calender 1 comprises a second cylinder 5 with a smooth surface. In the example both the cylinders 3 and 5 have an external surface made of steel.

The two cylinders 3 and 5 rotate in opposite directions with different speeds of rotation and, more particularly, the cylinder 3 equipped with protuberances 3A rotates at a peripheral speed which is greater than the peripheral speed of the cylinder 5. The difference between the two speeds of rotation is of the order of 15-50%. The film F is supplied to the calender 1 at a feeding speed which is less than the peripheral speed of the cylinder 3, for example at a speed equal to the peripheral speed of the cylinder 5, and in any case not greater than the speed of the cylinder 3. At the exit from the calender the film F' has a speed which substantially more or less corresponds to the peripheral speed of the cylinder 3 or is slightly greater.

At least one of the two cylinders 3 and 5 is heated and preferably both are heated to different temperatures which can be adjusted independently of one another. The temperature of the external surface of the cylinder 3 is preferably slightly higher than the temperature of the smooth cylinder 5. The surface temperature of the cylinders is higher than the softening temperature of the material forming the film F, but preferably lower than the melting temperature of the film itself. Moreover, the two cylinders 3, 5 are pressed against one another at high pressure. The surface temperature of the cylinders may be comprised for example between 70° and 240°C and the pressure between 120 and 220 kg/cm².

The arrangement is substantially equivalent to that described in EP-B-0,598,970.

Prior to entry into the calender 1, a web V consisting of unconsolidated fibers supplied by a production machine 7, for example a carding machine, is associated with the surface of the film F which is opposite to that which comes into contact with the cylinder 3 equipped with protuberances. The feeding speed of the web V is approximately equal to the feeding speed of the film F. Upstream of the calender 1, the web V and the overlying film F may be positioned on a support and travel surface or on a conveyor belt.

The web V consists of fibers which are unconsolidated, so as to obtain greater softness. The fibers have a length ranging, for example, between 30 and 60 mm and a count ranging, for example, between 1.7 and 25 dtex. The thickness of the web V of unconsolidated fibers ranges typically between 5 and 50 mm.

The film F and the web V arranged on top of one another, when passing into the nip between the cylinders 3 and 5, undergo varying transformations.

Firstly the film F is perforated owing to the combined effect of the temperature of the cylinders 3, 5, the pressure between the cylinders and the difference in peripheral speed between the cylinders and between the cylinder 3 and the film F. As already described in EP-B-0 598 970, the result of this combination of factors produces perforation of the film F with the formation of lugs of material which are partially detached from the base material, projecting from the opposite side to that making contact with the protuberances 3A of the cylinder 3. The film F when perforated (indicated by F') thus assumes a three-dimensional structure, in the sense that the overall thickness, determined by the thickness of the film added to the length of the lugs obtained as a result of the plastic deformation of the material, is much greater than the thickness of the non-perforated film and reaches a value of about ten times as much, passing for example from 30 to 300 µm or more.

Figs. 2, 3 and 4 schematically show the configuration of the perforated film F': P denotes the perforations and A the lugs of plastic material detached as a result of the plastic deformation of the base material. Fig. 2 also shows the surface of the film F' in contact with the cylinder 3: it has an embossed lozenge-shaped form which may be obtained during a prior machining operation involving embossing of the intact film F.

In addition to the perforation of the film F, adhesion of the web V of fibers to the perforated film F' is also obtained between the cylinders 3 and 5. Adhesion is obtained in the region of the perforations P, and more precisely in the region of the lugs A which are formed as a result of calendering of the film between the cylinders 3 and 5. In fact, in the region of the protuberances 3A of the cylinder 3, the combined effect of the temperature and the pressure causes localized melting of the plastic material which, detached from the base film, produces the lugs A and/or fibers forming the web V. The successive cooling of the combined film + web leaving the calender 1 causes solidification of the points of plastic material previously melted and consequently mutual adhesion between the web and the film.

The perforations are sufficiently close together, for example with a density of 30-90 perforations per cm² to ensure a high number of joining points between the web V and the perforated film F' and guarantee adhesion of the fibers forming the web V to the film even though the web itself is formed by unconsolidated fibers.

The product which is obtained is characterized, therefore, by a high degree of softness of the fibers of the web V which remain fairly adherent to the perforated film F', but loose and soft enough to perform their function of distribution of the liquid when the laminar material thus obtained is used for the manufacture of an absorbent article.

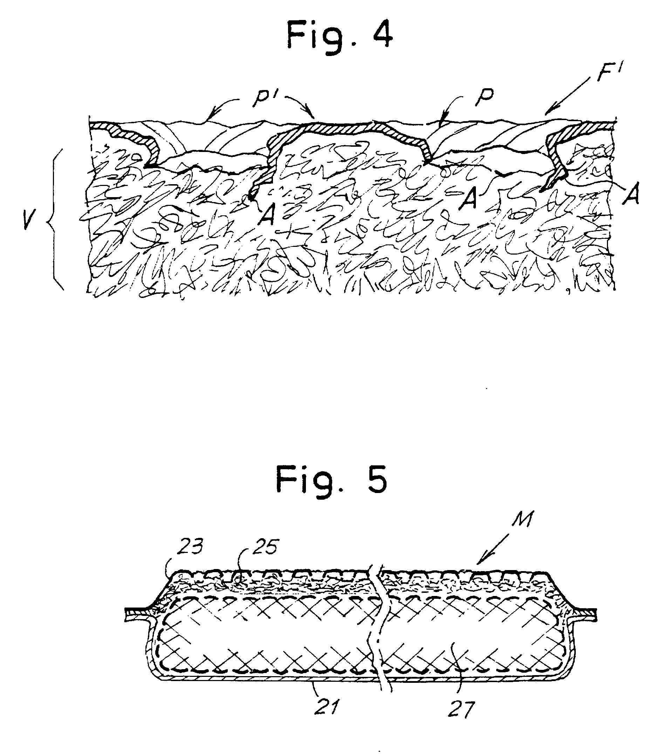

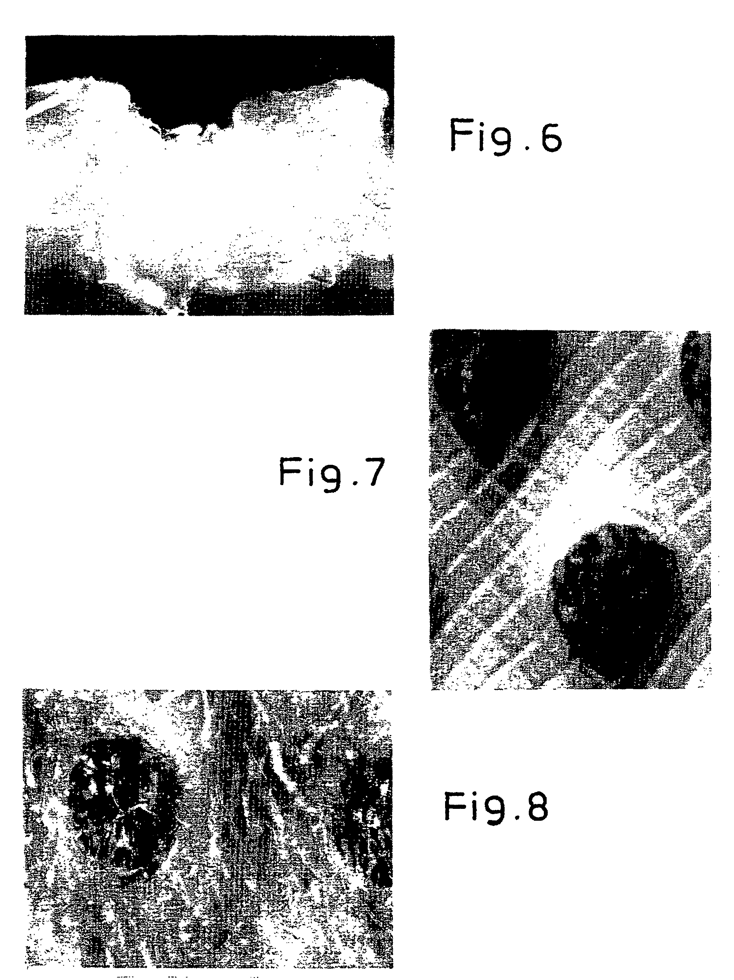

The structure of the laminar product composed of web + perforated film which is obtained is illustrated schematically in Figs. 2 and 4 and in its real state in the photographic enlargements of Figs. 6 to 8.

Fig. 5 shows the use of the laminar material thus obtained, in the production of an absorbent article M, for example a sanitary napkin, a diaper for children or the like. The article M has a back sheet 21, a top sheet 23 formed by a perforated film, an acquisition layer 25 and a central absorbent mass 27. The top sheet 23 and the acquisition layer consist of laminar material obtained with the method described above, the top sheet being formed by the perforated film F' and the acquisition layer being formed by the layer of fibers defining the web V applied to the film F'.

The film F is preferably a film consisting of several layers of different material, typically a film with three or five layers. The internal layer has typically a melting temperature which is higher than that of the external layers, so that during the perforation process only the external zone of the film is made to melt at the points where the protuberances 3A act. The internal layer of the film is, for example, a layer of linear low-density polyethylene (LLDPE), while the external layers consist of very low-density linear polyethylene (VLDLP) or ethyl vinyl acetate (EVA).

The fibers forming the web V are bi-component fibers or mixtures of single-component and bi-component fibers or else mixtures of fibers with two different melting points. In particular it is possible to use bi-component fibers with an external component forming a shell, which has a lower melting temperature than the internal component forming the core of the fiber. For example it is possible to use fibers with a core consisting of polypropylene having a melting temperature of 160°C, while the external shell is made of polyethylene with a melting temperature of 120°C. In this way, only the shell of the fibers in contact with the protuberances 3A of the cylinder 3 melts inside the calender 1.

In order to reduce further the quantity of fibers which reach the melting point inside the calender 1, it is possible to use mixtures formed by single-component polypropylene fibers, with a relatively high melting temperature, and bi-component fibers with a polypropylene core and polyethylene shell. In this way melting of the synthetic material forming the fibers is reached only on some of the fibers forming the web (the bi-component fibers) and limited only to the shell thereof. In this way the web V maintains its soft and voluminous structure even after calendering. These fibers are known to the persons skilled in the art.

The temperature of the surface of the cylinders 3 and 5 and the pressure with which they are pressed against one another are selected also according to the material forming the fibers so as to obtain a degree of melting which is adequate for ensuring mutual adhesion between the web V and the perforated film F', but sufficiently limited not to lose the softness of the web V.

It is understood that the drawing shows only one practical embodiment of the invention, the forms and arrangements of which may vary without, however, departing from the inventive idea.

## Claims

1. Method for the production of a three-dimensional perforated thermoplastic film, including the steps of sumperimposing an intact film (F) and a web of fibers (V) and perforating said film (F) causing, in the region of perforations (P) produced in the film, the mutual adhesion between said film and said web, **characterized in that** said web is a web of unconsolidated fibers, the fibers of said web remaining substantially free and soft after perforation.

2. Method as claimed in claim 1, **characterized by** the fact of maintaining a substantially uniform density of the fibers of said web.

3. Method as claimed in claim 1 or 2, **characterized by** the fact of perforating said film, forming lugs of plastic material (A) projecting from the plane of the film (F) and welding said web (V) to said film in the region of said lugs.

4. Method as claimed in claim 3, **characterized by** the fact of perforating said film by means of a pair of counter-rotating cylinders (3, 5) which are pressed against one another and one of which has a substantially smooth surface and the other a plurality of protuberances (3A), the cylinder equipped with protuberances rotating at a peripheral speed which is greater than the peripheral speed of the other cylinder and greater than the speed of feeding of the film (F) and web (V) between said cylinders, in correspondence of said protuberances the film being perforated with the formation of lugs (A) of material which are partially detached from said film and the fibers of said web being welded to said lugs.

5. Method as claimed in one or more of the preceding claims, **characterized in that** said fibers are thermoplastic fibers.

6. , Method as claimed in claim 5, **characterized in that** at least some of said fibers have a melting temperature which is lower than the melting temperature of said thermoplastic film.

7. Method as claimed in one or more of the preceding claims, **characterized by** the fact of forming said web of fibers with at least partly bi-component fibers, consisting of a shell and a core, the shell having a melting temperature lower than that of the core.

8. Method as claimed in claim 7, **characterized by** the fact of forming the web of fibers comprising partly bi-component fibers and partly single-component fibers, the single-component fibers having a melting temperature which is higher than the melting temperature of the shell of the bi-component fibers.

9. Method as claimed in one or more of the preceding claims, **characterized in that** said thermoplastic film has a layered structure, with at least one layer having a lower melting temperature and one layer having a higher melting temperature.

10. A composite laminar material comprising a three-dimensional thermoplastic film (F') equipped with perforations (P) and a web (V) of fibers joined by means of welding to said film in the region of said perforations, **characterized in that** said web is a web of unconsolidated, substantially free and soft fibers-.

11. Laminar material as claimed in claim 10, **characterized in that** lugs (A) of thermoplastic material forming the film, which are partially detached from the base material and project from the film, are provided around said perforations and **in that** said web of fibers is welded to said film in the region of said lugs.

12. Laminar material as claimed in claim 10 or 11, **characterized in that** it has a density of perforations of between 30 and 90 perforations per cm².

13. Laminar material as claimed in one or more of claims 10 to 12, **characterized in that** said web of fibers comprises at least partly bi-component fibers, consisting of a shell and a core, the shell having a melting temperature lower than that of the core.

14. Laminar material as claimed in claim 13, **characterized in that** said web of fibers comprises partly bi-component fibers and partly single-component fibers, the single-component fibers having a melting temperature higher than the melting temperature of the shell of the bi-component fibers.

15. Laminar material as claimed in one or more of claims 10 to 14, **characterized in that** said thermoplastic film has a layered structure, with at least one layer having a lower melting temperature and one layer having a higher melting temperature.

16. Absorbent article comprising an absorbent mass arranged between a non-permeable back sheet and a top sheet permeable to liquids and comprising, moreover, an acquisition layer arranged between said top sheet and said absorbent mass, **characterized in that** said top sheet consists of a laminar material as claimed in one or more of claims 10 to 15 and **in that** the web of fibers forms said acquisition layer.

## Patentansprüche

1. Verfahren zur Herstellung eines dreidimensionalen, perforierten, thermoplastischen Films, mit den Schritten:
Übereinanderlegen eines unversehrten Films (F) und einer Faserstoffbahn (V) und Perforieren des Films (F), wodurch im Bereich der in dem Film erzeugten Perforationen (P) eine gegenseitige Haftung des Films und der Faserstoffbahn bewirkt wird, **dadurch gekennzeichnet, daß** die Bahn eine Bahn aus lockeren Fasern ist, wobei die Fasern der Bahn nach der Perforation im wesentlichen frei und weich bleiben.

2. Verfahren nach Anspruch 1, **dadurch gekennzeichnet, daß** eine im wesentlichen gleichförmige Dichte der Fasern der Bahn aufrechterhalten wird.

3. Verfahren nach Anspruch 1 oder 2, **gekennzeichnet durch** Perforieren des Films, Ansätzen von Bildern aus Kunststoffmaterial (A), die an der Ebene des Films (F) vorstehen und Schweißen der Bahn (V) im Bereich der Ansätze an den Film.

4. Verfahren nach Anspruch 3, **dadurch gekennzeichnet, daß** der Film mittels eines Paares gegenläufig drehender Zylinder (3, 5) perforiert wird, die gegeneinander gepreßt werden, und von denen einer eine im wesentlichen glatte Oberfläche hat, und der andere eine Anzahl von Vorsprüngen (3A) hat, wobei der Zylinder, der mit den Vorsprüngen ausgerüstet ist, mit einer Umfangsgeschwindigkeit dreht, die größer als die Umfangsgeschwindigkeit des anderen Zylinders und größer als die Zuführgeschwindigkeit des Films (F) und der Bahn (V) zwischen die Zylinder ist, wobei der Film in Übereinstimmung mit den Vorsprüngen mit der Ausbildung von Ansätzen (A) aus Material perforiert wird, die teilweise von dem Film gelöst sind und die Fasern der Bahn mit den Ansätzen verschweißt werden.

5. Verfahren nach einem oder mehreren der vorstehenden Ansprüche, **dadurch gekennzeichnet, daß** die Fasern thermoplastische Fasern sind.

6. Verfahren nach Anspruch 5, **dadurch gekennzeichnet, daß** wenigstens einige der Fasem eine Schmelztemperatur haben, die niedriger als die Schmelztemperatur des thermoplastischen Films ist.

7. Verfahren nach einem oder mehreren vorstehenden Ansprüche, **dadurch gekennzeichnet, daß** die Faserstoffbahn wenigstens teilweise mit Bi-Komponentenfasern gebildet wird, bestehend aus einem Mantel und einem Kern, wobei der Mantel eine niedrigere Schmelztemperatur als der Kern hat.

8. Verfahren nach Anspruch 7, **dadurch gekennzeichnet, daß** die Faserstoffbahn, teilweise Bi-Komponentenfasern und teilweise Ein-Komponentenfasern enthält, wobei die Ein-Komponentenfasern eine Schmelztemperatur haben, die höher als die Schmelztemperatur des Mantels der Bi-Komponentenfasern ist.

9. Verfahren nach einem oder mehreren der vorstehenden Ansprüche, **dadurch gekennzeichnet, daß** der thermoplastische Film eine Schichtstruktur hat, wobei wenigstens eine Schicht eine niedrigere Schmelztemperatur hat und eine Schicht eine höhere Schmelztemperatur hat.

10. Laminares Verbundmaterial bestehend aus einem dreidimensionalen thermoplastischen Film (F'), der mit Perforationen (P) versehen ist und einer Bahn (V) aus Fasern, die mittels Schweißen im Bereich der Perforationen an den Film angeschweißt sind, **dadurch gekennzeichnet, daß** die Bahn eine Bahn aus losen, im wesentlichen freien und weichen Fasern ist.

11. Laminares Material nach Anspruch 10, **dadurch gekennzeichnet, daß** um die Perforationen Ansätze (A) aus dem thermoplastischen Material, welches den Film bildet, und die teilweise von dem Basismaterial gelöst sind und an dem Film vorstehen, vorgesehen sind und daß die Faserstoffbahn an den Film im Bereich dieser Ansätze angeschweißt ist.

12. Laminares Material nach Anspruch 10 oder 11, **dadurch gekennzeichnet, daß** es eine Perforationdichte zwischen 30 und 90 Perforationen pro cm² hat.

13. Laminares Material nach einem oder mehreren Ansprüche 10 bis 12, **dadurch gekennzeichnet, daß** die Faserstoffbahn wenigstens teilweise Bi-Komponentenfasern, bestehend aus einem Mantel und einem Kern hat, wobei der Mantel eine niedrigere Schmelztemperatur als der Kern hat.

14. Laminares Material nach Anspruch 13, **dadurch gekennzeichnet, daß** die Faserstoffbahn teilweise Bi-Komponentenfasern und teilweise Einzelkomponentenfasern aufweist, wobei die Ein-Komponentenfasem eine höhere Schmelztemperatur als die Schmelztemperatur des Mantels der Bi-Komponentenfasern haben.

15. Laminares Material nach einem oder mehreren der Ansprüche 10 bis 14, **dadurch gekennzeichnet, daß** der thermoplastische Film eine Schichtstruktur hat, wobei wenigstens eine Schicht eine niedrigere Schmelztemperatur und eine Schicht eine höhere Schmelztemperatur hat.

16. Absorbierender Artikel bestehend aus einer absorbierenden Masse, die zwischen einer nicht durchlässigen Rückseite und einer Oberseite, die für Flüssigkeiten durchlässig ist, angeordnet ist, und die darüber hinaus eine Aufnahmeschicht aufweist, die zwischen der Oberseite und der absorbierenden Masse angeordnet ist, **dadurch gekennzeichnet, daß** die Oberseite aus einem laminaren Material wie nach einem oder mehreren der Patentansprüche 10 bis 15 beansprucht besteht und daß die Faserstoffbahn die Aufnahmeschicht bildet.

## Revendications

1. Procédé de fabrication d'un film thermoplastique perforé tridimensionnel, comprenant les étapes consistant à superposer un film vierge (F) et un faisceau de fibres (V) et à perforer ledit film (F) ce qui amène, dans la région des perforations (P) réalisées dans le film, l'adhérence mutuelle entre ledit film et ledit faisceau de fibres, **caractérisé en ce que** ledit faisceau de fibres est un faisceau de fibres non consolidées, les fibres dudit faisceau demeurant sensiblement libres et souples après perforation.

2. Procédé selon la revendication 1, **caractérisé par** le fait de maintenir une densité sensiblement uniforme des fibres dudit faisceau.

3. Procédé selon la revendication 1 ou 2, **caractérisé par** le fait de perforer ledit film, de former des pattes d'un matériau en plastique (A) se projetant à partir du plan du film (F) et à souder ledit faisceau (V) au dit film dans la région desdites pattes.

4. Procédé selon la revendication 3, **caractérisé par** le fait de perforer ledit film au moyen d'une paire de cylindres tournant en sens contraire (3, 5) qui sont pressés l'un contre l'autre et dont l'un d'entre eux a une surface sensiblement régulière, et dont l'autre présente une pluralité de protubérances (3A), le cylindre muni des protubérances tournant à une vitesse périphérique qui est plus élevée que la vitesse périphérique de l'autre cylindre, et plus élevée que la vitesse d'alimentation du film (F) et du faisceau (V) entre lesdits cylindres ; en correspondance avec lesdites protubérances, le film étant perforé avec la formation de pattes (A) de matériau qui sont partiellement détachées à partir dudit film, et les fibres dudit faisceau étant soudées aux dites pattes.

5. Procédé selon une ou plusieurs des revendications précédentes, **caractérisé en ce que** lesdites fibres sont des fibres thermoplastiques.

6. Procédé selon la revendication 5, **caractérisé en ce que** au moins quelques-unes desdites fibres ont une température de fusion qui est plus basse que la température de fusion dudit film thermoplastique.

7. Procédé selon une ou plusieurs des revendications précédentes, **caractérisé par** le fait de réaliser ledit faisceau de fibres avec au moins en partie des fibres à deux composants comprenant une enveloppe et un coeur, l'enveloppe ayant une température de fusion plus basse que celle du coeur.

8. Procédé selon la revendication 7, **caractérisé par** le fait de réaliser le faisceau de fibres comprenant en partie des fibres à deux composants et en partie des fibres à un seul composant, les fibres à un seul composant ayant une température de fusion qui est plus élevée que la température de fusion de l'enveloppe des fibres à deux composants.

9. Procédé selon une ou plusieurs des revendications précédentes, **caractérisé en ce que** ledit film thermoplastique a une structure en couches, avec au moins une couche ayant une température de fusion moins élevée et une couche ayant une température de fusion plus élevée.

10. Matériau composite laminaire comprenant un film thermoplastique tridimensionnel (F') muni de perforations (P) et d'un faisceau (V) de fibres assemblé à celui-ci au moyen du soudage dudit film dans la région desdites perforations, **caractérisé en ce que** ledit faisceau est un faisceau de fibres non consolidées, sensiblement libres et souples.

11. Matériau laminaire selon la revendication 10, **caractérisé en ce que** des pattes (A) de matériau thermoplastique formant le film, qui sont partiellement détachées du matériau de base et qui se projettent à partir du film, sont prévues autour desdites perforations, et **en ce que** ledit faisceau de fibres est soudé au dit film dans la région desdites pattes.

12. Matériau laminaire selon la revendication 10 ou 11, **caractérisé en ce qu'**il a une densité de perforations comprise entre 30 et 90 perforations au cm².

13. Matériau laminaire selon une ou plusieurs des revendications 10 à 12, **caractérisé en ce que** ledit faisceau de fibres comprend au moins en partie des fibres des fibres à deux composants, comprenant une enveloppe et un coeur, l'enveloppe ayant une température de fusion plus basse que celle du coeur.

14. Matériau laminaire selon la revendication 13, **caractérisé en ce que** ledit faisceau de fibres comprend en partie des fibres des fibres à deux composants et en partie des fibres à un seul composant, les fibres à un seul composant ayant une température de fusion qui est plus élevée que la température de fusion de l'enveloppe des fibres à deux composants.

15. Matériau laminaire selon une ou plusieurs des revendications 10 à 14, **caractérisé en ce que** ledit film thermoplastique a une structure en couches, avec au moins une couche ayant une température de fusion moins élevée et une couche ayant une température de fusion plus élevée.

16. Article absorbant comprenant une masse absorbante disposée entre une plaque d'appui non perméable et une plaque supérieure perméable aux liquides, et comprenant en outre une couche d'acquisition disposée entre ladite plaque supérieure et ladite masse absorbante, **caractérisé en ce que** ladite plaque supérieure se compose d'un matériau laminaire selon une ou plusieurs des revendications 10 à 15 et **en ce que** le faisceau de fibres forme ladite couche d'acquisition.
